# EUROPEAN PATENT SPECIFICATION

(11) **EP 1 313 425 B1**
(45) Date of publication and mention of the grant of the patent: **07.03.2007**
(21) Application number: 01960172.3
(22) Date of filing: 12.07.2001
(51) Int. Cl.: A61J 3/00

(54) **METHOD OF MOULDING A PHARMACEUTICAL COMPOSITION IN A PACKAGING MATERIAL**
VERFAHREN ZUM FORMEN EINER PHARMAZEUTISCHEN ZUSAMMENSETZUNG IN EINEM VERPACKUNGSMATERIAL
PROCEDE POUR MOULER UNE COMPOSITION PHARMACEUTIQUE DANS UN MATERIAU D'EMBALLAGE

(30) Priority: 14.07.2000 DK 200001097; 08.08.2000 US 223802 P
(43) Date of publication of application: 28.05.2003
(73) Proprietor: NOVO NORDISK A/S, 2880 Bagsvaerd (DK)
(72) Inventor: BUCH-RASMUSSEN, Thomas, DK-2820 Gentofte (DK); AASMUL, Sören, DK-2840 Holte (DK); POULSEN, Jens-Ulrik, DK-2830 Virum (DK); FLINK, James, M., DK-2930 Klampenborg (DK); HANSEN, Philip, DK-2840 Holte (DK); JUUL-MORTENSEN, Claus, DK-2000 Frederiksberg (DK)
(74) Representative: Hansen, Einar Tronier
(86) International application number: PCT/DK2001/000489
(87) International publication number: WO 2002/005744

(56) References cited:
- EP-A1- 0 838 209
- US-A- 4 153 406
- US-A- 4 920 727
- US-A- 6 098 675

## Description

The present invention relates to a method of producing a pharmaceutical composition in a packaging material, a moulded packaging material comprising the pharmaceutical composition and a cassette comprising the packaging material.

### Background of the invention

Some drugs are administered parenterally, either because a rapid effect is desired, or because due to the nature of the drug it will be destroyed in the stomach before any effect of the drug has occurred.

By far the most widely used method for parenteral injection of drugs is by injection of an aqueous solution using a hypodermic syringe. The use of aqueous solutions is associated with a number of inherent problems. In order to inject a given volume of drug, a much larger volume of water and different additives also have to be injected, leading to injection of a high volume. In particular for muscular injection the pain associated with injection is primarily caused by the volume injected, not by the penetration of the skin. Any reduction in volume would thus lead to a reduction in pain for the patient.

Injection of drugs as solid particles have been discussed in the prior art, such as powder injection and injection of pharmaceutical compositions having the shape of needles, the latter being administerable with greater precision than the powders.

Injection moulding of tablets has been attempted which is described for example by Cuff et Raouf in "A Preliminary Evaluation of Injection Moulding as a Technology To Produce Tablets. The production of mouldable compositions for injection purposes has not been suggested yet.

To obtain a satisfactory solid-dose-parenteral-injection, the pharmaceutical composition to be injected must be of small volume to avoid injection pain and to achieve a desired dissolution rate. Also, preferably the pharmaceutical composition should be provided with a well-defined strength to make it possible to penetrate the cutis of the patient, be it an animal or human being. Furthermore, the pharmaceutical composition should be long-term stable at ambient temperature in terms of both strength and structure of the pharmaceutical composition and the biological activity of the drug. The pre-determined strength may be provided by using a carrier in addition to the drug to be administered. A carrier used to provide the necessary strength should comprise compounds that are tissue compatible and that are contained in the pharmacopoeia.

Injectable pharmaceutical compositions are by nature very small in order to be injected essentially without causing pain. A problem with these compositions is however their size, since such sizes cannot be handled without a tool like a pincer, to arrange the composition for injection.

It is known, e.g. from US 6098675 or EP 0838209, to shape a pharmaceutical composition before inserting it in a packaging material.

### Summary of the invention

By the present invention the pharmaceutical composition is moulded directly into the packaging material thereby obviating any needs for transporting the delicate structures after moulding.

Thus, the present invention relates to a method of producing a pharmaceutical composition in a packaging material comprising the steps of
establishing a mould,
moulding, in said mould, the packaging material from a packaging melt, whereby the packaging material is provided with at least one cavity, said cavity having at least one side wall and optionally a bottom wall,
moulding in a moulding compartment the pharmaceutical composition from a pharmaceutical melt consisting of the ingredients of the pharmaceutical composition having a predetermined temperature, wherein said moulding compartment having a predetermined form being defined by at least a part of said at least one cavity,
cooling the pharmaceutical composition,
obtaining in the packaging material the pharmaceutical composition having a predetermined form.

By the present method it is possible to provide a method for preparing a pharmaceutical composition being injectable without any uses of transfer needles or the like whereby the composition is capable of penetrating the epidermis or the mucosa of an individual by itself. However the pharmaceutical composition produced in accordance with this invention may of course also be implanted or injected by use of transfer needles and/or trocars.

The packaging material defines the predetermined geometry of the pharmaceutical composition, in that a melt of the pharmaceutical composition is injected directly into the packaging material.

In another embodiment the invention relates to a moulded packaging material comprising at least one cavity, said cavity having at least one side wall and optionally a bottom wall, and a pharmaceutical composition being moulded in at least a part of said at least one cavity from a melt consisting of the ingredients of the pharmaceutical composition.

In particular the packaging material comprises at least one cavity, said cavity having at least one side wall and optionally a bottom wall, and a pharmaceutical composition being moulded in at least a part of said at least one cavity from a melt consisting of the ingredients of the pharmaceutical composition, whereby the pharmaceutical composition is in contact with the cavity side wall in an area being more than half of the surface area of the pharmaceutical composition.

The pharmaceutical composition is preferably in contact with the cavity side wall in an area being more than half of the surface of the pharmaceutical composition to ensure that the direction of the pharmaceutical composition in the packaging material is optimal for ejection without damages to the pharmaceutical composition.

By the term in contact with means that substantially no movements of the pharmaceutical composition in the packaging material in a plane perpendicular to the longitudinal axis is possible.

Yet a further aspect relates to a cassette comprising a packaging material as defined above and furthermore comprising ejecting means for ejecting the pharmaceutical composition from the cavity.

A dosage of therapeutic agent comprised in a pharmaceutical composition can be administered to a patient in need thereof from a cassette as defined above, comprising
- arranging the cassette adjacent the skin of the patient,
- activating an ejecting means,
- injecting the pharmaceutical composition comprising the therapeutic agent.

### Drawings

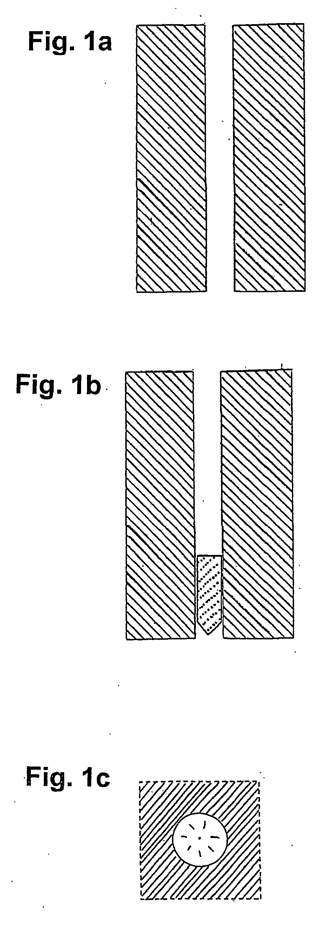

Fig. 1 a shows a vertical cross section through a moulded packaging material prior to moulding the pharmaceutical composition, Fig. 1 b shows a vertical cross section through a moulded packaging material comprising the pharmaceutical composition, and Fig. 1 c shows a horizontal cross section through a moulded packaging material comprising the pharmaceutical composition.
Fig. 2: In one configuration, the packaging material is formed with a further cavity which fit to an inserter (6). The inserter and the further cavity are constructed with the aim to be able to insert the pharmaceutical composition into human subcutanous tissue. Moving the inserter forward, the inserter presses the pharmaceutical composition (3) through the protective membrane (9) and into the human skin. The movement will be stopped when the inserter head (8) hits the container (1). At this end position the inserter head (5) will stick approximatively 1 mm out from the container and into the human skin. The pharmaceutical composition will be in front of the inserter and thus be in the subcutaneous tissue. The inserter can be fitted to the container in such a way, that after insertion the inserter is withdrawn and thus free from the skin.

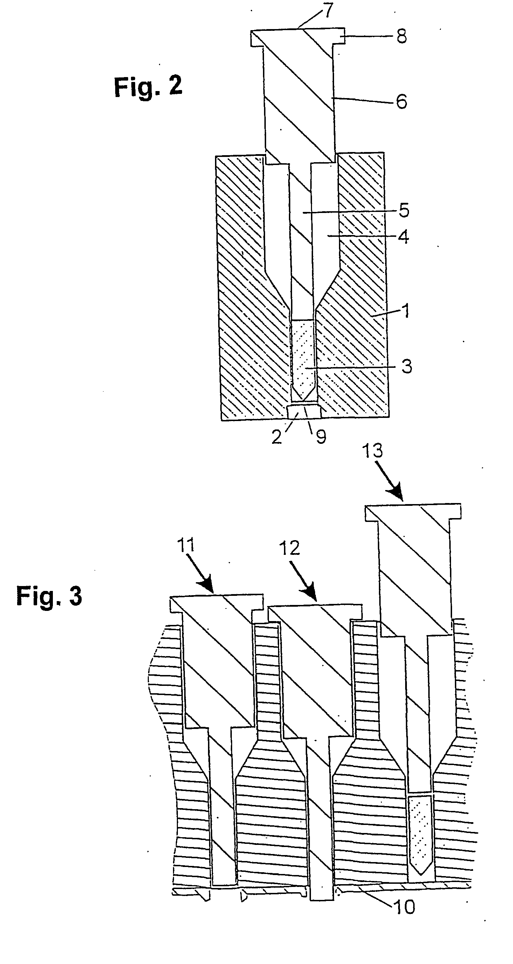

Fig. 3: From the initial position of the inserter (13), the inserter is pressed forward to the end position (12) and automatically withdrawn to position (11).

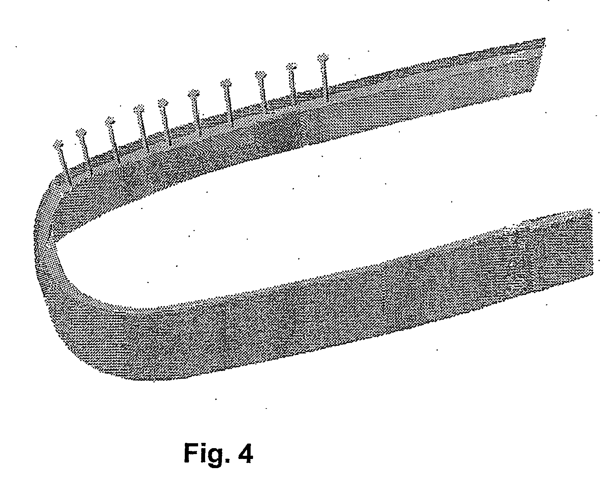

Fig. 4: The packaging container, the pharmaceutical composition and the inserter can be a single unit or a multiple unit. In this figure the system is formed as a cassette where the container part is formed in one moulding stem and the pharmaceutical composition in a second moulding step.

### Detailed description of the invention

The packaging material is described in the following as a single unit of packaging material having a cavity for housing the pharmaceutical composition, but as described below the packaging material may comprise several cavities each housing an individual pharmaceutical composition.

The packaging material may serve at least two functions:
- Serving as a mould for the pharmaceutical composition
- Serving as packaging when storing the moulded pharmaceutical composition.

Also the packaging material may serve as a cassette or a part of a cassette for administering the pharmaceutical composition.

In the present context "ejection" means the action of forcing the pharmaceutical composition out of the packaging material.

The term "administering" is used in its normal meaning, i.e. to press or inject the pharmaceutical composition into the patient in need thereof, such as an animal or a human being.

The administration may be conducted through the use of a transfer needle or by injecting the composition directly through the skin without the use of a transfer needle, wherein the latter is preferred.

The packaging material is moulded in a mould defining the outer shapes of the packaging material. Furthermore, during moulding the packaging material is provided with a cavity, the cavity being positioned in the packaging material so that access to the cavity is possible from the outside.

In a preferred embodiment a mould comprising an inserter is provided, said inserter having a shape corresponding to the predetermined shape of the cavity. The packaging melt is then injected into the mould comprising the inserter, and after having packed the mould the melt is cooled and the inserter is removed.

The inserter may be in one or several parts depending on the predetermined geometry of the cavity.

Accordingly, the packaging material is provided with at least one cavity, said cavity having at least one side wall and optionally a bottom wall as discussed below.

The cavity is provided with one side wall when having a round, such as circular or oval cross-section. The cavity may be provided with 3, 4, 5 or more side walls, when the cross-section is triangular, square, rectangular, pentangular, or higher. It is preferred, that the cavity is substantially round.

Before moulding the pharmaceutical composition the cavity may be lined with another material, such as a lubricant. It is however preferred that the cavity side wall is defined by the packaging material.

The cavity defined within the packaging material is preferably having a volume larger than the pharmaceutical composition to be moulded therein. Thereby for example an injection head may be guided by the cavity when injecting the melt for the pharmaceutical composition.

The cavity is preferably an elongated cavity, such a through bore, having two open ends. In a preferred embodiment however the cavity comprises a bottom wall moulded from packaging melt. The bottom wall may then function as a diffusion tight sealing of one end of the cavity during storage.

In an alternative embodiment the cavity is provided with a top wall as well, whereby access to the cavity may be obtained for example through an opening in the side wall.

The moulding compartment for the pharmaceutical composition constitutes at least a part of the cavity, or the whole cavity.

The moulding compartment has a predetermined form, that is defined by the cavity side walls, the cavity bottom, that may be a mould part or packaging material as discussed above and an insertion means, said insertion means being inserted into the cavity through an opening of the cavity.

The insertion means preferably comprises an injection head for injecting the pharmaceutical composition melt into the moulding compartment. During moulding of the pharmaceutical composition the insertion means may be arranged adjacent to or in the open end of the cavity and pharmaceutical melt is injected into the moulding compartment and packed.

The injection head may however also be arranged to inject through an opening in the side wall of the cavity, whereby the insertion means simply constitutes a part of the moulding compartment.

The moulding compartment is defining the form of the pharmaceutical composition, and is preferably elongated, whereby the predetermined form of the pharmaceutical composition is preferably an elongated form.

The moulding compartment is furthermore preferably so defined that one end of the elongated moulded pharmaceutical composition will become plane during moulding. The plane end obtained is capable of transferring a more even pressure from the ejection means to the remaining part of the pharmaceutical composition during ejection, reducing the risk of damaging the pharmaceutical composition during ejection.

In principle the packaging material may assume any form suitable for functioning as at least a part of a mould for moulding the pharmaceutical composition.

For use with therapeutic agents for which only one dose is needed, such as glucagon, adrenaline, or atropine, a single unit packaging may be used, whereas for injection of medicine administered frequently, such as insulin or growth hormone, an array of more units is preferred. In the present context the term "unit" means cavities in the packaging material. The units may be arranged in line in a beam shaped packaging material or in a revolver drum shaped packaging material or a number of single packaging material each comprising one unit may be designed to be hinged together to form a cartridge belt. The choice of design may depend on the number of units of pharmaceutical compositions to be stored in the packaging. When using several units it is preferred that the units are moulded simultaneously in a mould comprising a compartment for each unit, such as at least 5 cavities arranged in parallel, said cavities being substantially identical. When moulding individual packaging material these may be assembled subsequently through for example hinging means.

The packaging material will store the moulded pharmaceutical composition from moulding to administration. The pharmaceutical composition is preferably administered by ejection or pressing it from the packaging.

In order for the packaging material to serve as a part of a cassette for administering the pharmaceutical composition the packaging material must be sufficiently rigid, so that the pressing force applied to the pharmaceutical composition is not merely absorbed by the packaging material, but may be used to eject the pharmaceutical composition.

Any suitable pharmaceutical packaging material capable of being moulded, such as injection moulded may be used. The packaging material is preferably moulded from a polymer melt, such as a polymer selected from a polyolefin, a cyclic olefin, a cyclic polyolefin.

Examples of polymers may be polycarbonate, polystyrene, polyesters, polyethylene naphtalate, polyfluor fluorethylene propylene (FEP), such as fluor ethylene hexafluor propylene copolymer.

Other examples are poly(ethylene terephtalate) (PET), poly(ethylene naphtalate) (PEN), and acrylonitrile butadiene styrene terpolymer (ABS).

Any suitable moulding techniques known to the person skilled in the art may be used, such as injection moulding.

It is important the packaging material maintains its shape after moulding and during storage, since this would otherwise impair the function of the cavity with respect to moulding the pharmaceutical composition and later ejecting the composition.

Therefore substantially no shrinkage or contraction of the packaging material is allowed after moulding the pharmaceutical composition therein. Thus, it is preferred to cool the packaging material after moulding and before injection of the pharmaceutical melt, thereby allowing any contraction due to temperature changes to take place before production of the pharmaceutical composition.

Substantially no contraction means that the moulded packaging material contracts at most 5 % after the step of moulding the pharmaceutical composition, preferably at most 3 %, more preferably at most 1 %. The most important contractions with respect to the functions of the packaging material are those in a plane perpendicular to the longitudinal axis of the elongated cavity.

The production method may be a sequential two-component moulding, whereby the pharmaceutical composition is moulded in the cavity immediately after the moulding of the packaging material, optionally while the packaging material is remaining in the mould. In between the two moulding steps may be a cooling step if necessary. Preferably, the pharmaceutical composition is moulded at most 60 sec. after moulding the packaging material.

The packaging material is preferably cooled to a temperature substantially identical to the predetermined temperature of the pharmaceutical melt. Thereby a too fast freezing of the pharmaceutical melt in the cavity is avoided during moulding of the pharmaceutical composition.

It is furthermore preferred that no melting or softening of the packaging material is allowed to happen when the pharmaceutical melt is injected into the cavity. Accordingly, it is preferred that the glass transition temperature (Tg) of the packaging material is at most 20 °C lower than the predetermined temperature of the pharmaceutical melt, more preferred the Tg is identical to or higher than the predetermined temperature.

The glass transition temperature (Tg) of the packaging material is preferably above 100 °C, such as above 120 °C, preferably at least 140 °C or higher.

Contraction however may also be due to a phase shift, i.e. after-crystallisation of the packaging material. This is preferably overcome by using a moulded packaging material being an amorphous material, such as an amorphous polymer.

Crystalline polymers may also be used when the time necessary for crystallisation and contraction is accounted for before moulding the pharmaceutical composition.

The amorphous polymer may be a cyclic olefin copolymer, such as an olefin comprising a 5 or 7 membered ring optionally copolymerised with a linear olefin, such as polyethylene, polypropylene, and/or polybutylene.

In a preferred embodiment the packaging material is a 5 to 7 membered bicyclic hydrocarbon copolymerised with ethylene, such as TOPAS ® from Schott.

The production method may also be conducted as an insert moulding process. By an insert moulding process is meant that two separate moulding steps are conducted, first the packaging material is moulded, and the obtained packaging material may then be stored for a time period before the pharmaceutical composition is moulded in the packaging material. The requirements to the material for the packaging material are less demanding for an insert moulding process than for a two-component moulding process, and therefore an insert moulding process is a more flexible process. For example crystalline polymers may be used since the crystallisation is finished at least during storage of the packaging material before moulding the pharmaceutical composition.

In insert moulding the packaging material may be stored in the mould or the packaging material may be removed from the mould. In any situations the pharmaceutical composition is moulded into the cavity of the packaging material. The packaging material may be heated appropriately, for example to the predetermined temperature before injecting the melt comprising the ingredients for the pharmaceutical composition.

Whether the production is carried out as a sequential two-component moulding or an insert moulding is often merely a matter of choice, however in particular when producing a packaging material comprising more than one cavity the insert moulding may be a first choice.

Pharmaceutical compositions may be moulded in several cavities simultaneously when using a packaging material having more than one cavity, such as simultaneous moulding in at least 5 cavities, such as simultaneous moulding in at least 10 cavities or at least 15 cavities.

In a preferred embodiment the elongated pharmaceutical composition is moulded with a pointed end or a tip, whereby the administration of the composition in a substantially painless manner is facilitated. The pointed end or the tip is preferably defined during moulding by a tip mould insert arranged in an open end of the cavity. In the present context the term "pointed end" is used synonymously with the term "tip".

After moulding the tip mould insert may be removed to allow the composition to be injected.

The tip mould insert may be part of the insertion means comprising the injection head, it is however preferred that the tip mould insert is arranged in the end of the cavity opposite the insertion means comprising the injection head.

In case the cavity is provided with a bottom wall, the bottom wall may be shaped in a form corresponding to the tip, so that the tip is defined by the bottom wall. In this embodiment the bottom wall is preferably supported to the right shape by for example the mould. In this embodiment the bottom wall may be supported by the mould during injection of the pharmaceutical melt, to obtain the exact tip geometry.

In another embodiment the pharmaceutical composition is administrated through the use of a transfer needle or trocar whereby the transfer needle or trocar may be attached to the packaging material or loosely connected, so that the composition is injected from cavity via the needle or device through the skin or mucosa.

The cavity is preferably sealed, such as sealed with a diffusion tight sealing, during storage. The sealing may be applied after moulding the pharmaceutical composition or moulded with the packaging material.

The sealing may be any membrane providing the properties mentioned above. In one embodiment sealing of the ends is provided after moulding. Such as wherein the sealing of one end is a pin or a plug designed to seal the end when inserted into the cavity, whereby the pin or plug may also function as an anvil for ejecting the pharmaceutical composition. Thus, the method further comprises moulding a pin being dimensioned to fit into the open end of the cavity after retracting the insertion means. The pin is preferably moulded simultaneously with the packaging material, and more preferred from the same material.

As an alternative to the pin or in addition to and opposite to the pin another type sealing is preferably moulded from the packaging material, such as a membrane of packaging material constituting a bottom wall.

The sealing is preferably capable of being penetrated by the pharmaceutical composition during ejection, preferably without making any hindrance to the ejection route.

The pharmaceutical composition is moulded from a melt consisting of the ingredients of the pharmaceutical composition, such as at least one therapeutic agent and optionally at least one binder, capable of forming a melt and being injection moulded without compromising the ingredients.

When used the binder is present in an amount sufficient to produce, in combination with the therapeutic agent, the required strength of the composition. Therefore, the binder may constitute at least 1% by weight of the composition, such as at least 5% by weight of the composition. The upper limit of binder is mostly at most 90% by weight of the composition, such as at most 85%, such as at most 80%, such as at most 75%, such as at most 60%, such as at most 50%. Mostly the binder may constitute at least 5% and at most 80%, such as 60% by weight of the composition.

According to the invention, a solid pharmaceutical composition for parenteral injection having sufficient strength for parenteral injection yet having a relevant content of therapeutic agent is provided. The binder may be any kind of binder being mouldable and pharmaceutically acceptable, in particular acceptable for parenteral injection. The binder is preferably a polymer or a sugar or a sugar alcohol.

In addition to the strength, the binder may also provide the composition with a very smooth surface. Thereby, the friction upon penetration of the epidermis or mucosa is greatly reduced and consequently less force is required for the penetration and the penetration causes less pain. An advantage is that the composition is very stable both in terms of the biological activity of the therapeutic agent and in terms of the geometry and the strength of the composition even when it is stored at ambient temperature. Thereby, the invention combines compactness with ease of storage.

The binder is preferably a carbohydrate capable of providing an amorphous matrix, for example by adding a non-crystallisation agent to the binder.

According to an especially preferred embodiment, the binder essentially remains an amorphous matrix for at least 6 months at ambient temperature. This is achieved by carefully selecting the binding agent and the optional non-crystallisation agent so that the binding agent does not crystallise during storage. If the binding agent starts crystallising the composition will lose its strength, or if the crystallisation only takes place at the surface, the geometry of the composition will change and the friction upon injection will increase undesirably.

By the term strength is meant that the composition has sufficient compressive strength to penetrate the skin of a patient. It has been determined experimentally that a pressure force of at least 0.7 Newton is required to penetrate the epidermis of a human being with the claimed composition. Less is required to penetrate the mucosa. Consequently, the composition must be able to withstand such pressure force.

The strength can be tested in a force gauge tester such as an Advanced Force Gauge AFG-250N from Mecmesin, UK. Tests are carried out by formulating the composition as a rod and applying a pressure force to the rod. The pressure force is increased until the rod breaks. The instrument records the pressure force necessary to break the rod. This parameter is termed the compressive strength and should be understood as the breaking strength under compression.

Furthermore, at least 95 % of the strength of the composition should be maintained after 6 months, preferably after 12 months, at ambient temperature. It is important that the compositions are long term stable not only with respect to the biological activity and the structure of the composition, but also that the strength is essentially unaffected by storage. Some binding agents have a propensity to slowly crystallise after the amorphous glass matrix has been formed. Such binding agents are unsuitable for the present invention.

Preferentially, the composition should be essentially free from entrapped air. It is very important for the strength of the composition that no air is trapped inside the composition during processing in order to prevent air in the composition after cooling. Apart from reducing the strength, entrapped air also takes up unnecessary space and thereby reduces the amount of therapeutic agent contained in the composition.

As described above the composition is preferentially elongated having a cross section, which is substantially cylindrical, triangular, square, or polygonal. According to an especially preferred embodiment, the composition has the shape of a rod essentially cylindrical and pointed at one end. With compositions according to these embodiments, it becomes especially important to withstand crystallisation.

Many compounds are capable of forming a glass upon melting and subsequent quenching to below the glass transition temperature of the compound, Tg. Glasses can also be formed by dissolution and subsequent removal of the solvent, whereby the Tg is raised to above the storage and usage temperature. However, most compounds have a propensity to crystallise by themselves. Whereas an amorphous glass matrix often has a high compressive strength and a smooth surface, the same compound in a crystalline state has very limited compressive strength and a rough surface. The present compositions can be made from pure maltose or from pure sorbitol. These compounds will form glasses, but the compounds crystallise gradually at room temperature causing the strength to be reduced and the geometry to be changed. By mixing two or more compounds, crystallisation can be prevented or retarded.

The compositions are not necessarily completely water free. However, the water content of the binder is less than 20 % (w/w), preferably less than 10 %, more preferably less than 5 %, such as from 0.1 to 5 %, preferably from 1 to 5%. It has been determined that by having a water content between 0.1 and 5 %, the composition is not sticky and the binder is dissolved rapidly once administered. By lowering the water content even further, the rate of dissolution upon contact with the body fluids may be reduced disproportionately causing the therapeutic agent to be released only very slowly. Furthermore, many therapeutic agents such as proteins, peptides and polypeptides are more stable at a low water content than when completely dry. The advantage of having a low water content is that the therapeutic agent becomes biologically very stable and does not require special storage conditions such as refrigeration to maintain the biological activity. A third advantage is that the composition becomes essentially resistant to microbial attack, since microbes require a certain water content in order to establish a colony. Thus the requirement for handling the compositions become less rigid since the presence of a few microbes on the composition will not result in microbic proliferation and thereby not cause contamination. Finally the presence of excess water in the composition may result in water vapour during processing, which may give rise to air entrapment in the composition during subsequent cooling.

Several compounds may be used as the at least one binding agent and the invention is not limited to any specific compounds. According to a preferred embodiment the at least one binding agent is a mono-, di-, or oligosaccharide or a corresponding sugar alcohol or a derivative thereof. Many of these compounds are frequently used for pharmaceutical composition, are contained in the pharmacopoeia and can therefore readily be approved by the authorities. Furthermore, these compounds make an amorphous glassy matrix readily.

Furthermore, the at least one binding agent may be a carbohydrate-derivative. As mentioned, carbohydrates make amorphous glassy matrices readily. In some cases it is preferred to provide the composition with a binder having a slow rate of dissolution compared to binder made from true carbohydrates. This can be obtained by derivatising the carbohydrate, especially by adding non-polar groups to the carbohydrate, whereby the compound is rendered more hydrophobic.

According to a preferred embodiment the at least one binding agent is selected from maltose, sucrose, lactose, cellobiose, trehalose, maltulose, iso-maltulose, maltitol, sorbitol, Xylitol, mannitol, glucose, fructose, raffinose, melezitose, dextran, mannose, sorbose, melibiose, sophrose, turanose, lactulose, stachyose. This group of carbohydrates has excellent amorphous glass matrix making abilities. Furthermore, the carbohydrates are well known and can be purchased at reasonable price and in well characterised grades.

The optional at least one non-crystallisation agent may preferentially also be a carbohydrate, said carbohydrate being different from the binding agent. Likewise, the non-crystallisation agent may be a mono-, di-, or oligosaccharide, a corresponding sugar alcohol, or a derivative. It may be a natural or synthetic carbohydrate and according to an especially preferred embodiment the at least one non-crystallisation agent is selected from maltose, sucrose, lactose, cellobiose, trehalose, maltulose, iso-maltulose, maltitol, sorbitol, Xylitol, mannitol, glucose, fructose, raffinose, melezitose, dextran, mannose, sorbose, melibiose, sophrose, turanose, lactulose, stachyose.

Each combination of binding agent and non-crystallisation agent gives a unique amorphous glass matrix with a unique glass transition temperature, unique solubility, and unique strength. The composition has been found to perform excellently when the binding agent is selected from maltitol, sucrose, sorbitol, and mannitol and the non-crystallisation agent is selected from sorbitol, maltitol, and mannitol. These compounds are often used for pharmaceutical compositions, they all have the advantage of being edible and without any side effects upon administration.

According to an especially preferred embodiment, the binding agent is maltitol and the non-crystallisation agent is sorbitol and/or hydrogenated oligosaccharides. By using these specific compounds to make up the binder, especially excellent results are obtained since the obtained amorphous glass matrix has an optimal glass transition temperature and since the propensity to crystallise is very low. Furthermore, maltitol can be obtained in quantities and in a very suitable grade. Commercial maltitol is made by enzymatically degrading starch whereby a mixture of glucose, maltose, maltotriose and higher saccharides are formed. These are hydrogenated to form their corresponding sugar alcohols sorbitol, maltitol and hydrogenated oligosaccharides. Thus, the product contains primarily maltitol and sufficient amounts of the other sugar alcohols to prevent the crystallisation. Maltitol is tissue compatible, it is a well tested compound and has been used for years in the production of so-called sugarfree candies.

The binder comprising the at least one carbohydrate and the optional at least one non-crystallisation agent should not reduce the stability of the therapeutic agent. This could for instance take place via chemical reactions between the therapeutic agent and the components of the binder, either during processing or during storage. To avoid undesired reactions between aldehyde groups in reducing sugars and side chains of proteins, peptides, or polypeptides, the at least one carbohydrate and the at least one non-crystallisation agent are preferentially chosen from the group of non-reducing sugars.

When using a glassy binder the Tg of the binder in the final composition should preferably be at least 30°C. The Tg of the binder should be above ambient temperature, preferably 5 to 10°C above ambient temperature, or the composition will gradually melt during storage. Under certain special conditions, it may be necessary to select a binder having a higher Tg, e.g. for use in the tropics. With certain very heat labile therapeutic agents, it may be necessary to select a binder having a much lower Tg, so that the composition can be processed at e.g. 50°C. With such a low Tg, it may be necessary to store the compositions cold at 5°C and to inject them before the temperature rises above the Tg.

The invention is not limited by an upper Tg of the binder. Binders having a Tg from 40 to 120°C are preferred. Depending on the therapeutic agent, it is preferred that the Tg of the binder is less than 90°C, more preferably less than 80°C. A majority of therapeutic agent are heat labile and although many proteins or peptides can tolerate exposure to elevated temperatures in a dry state a loss of activity may nevertheless be encountered during processing. To reduce exposure of the therapeutic agent to elevated temperatures it is therefore preferable to select binders with a low Tg with due respect to the lower limits mentioned above.

When using a polymeric binder the melting temperature of the binder is preferably within the intervals mentioned above for Tg of the glassy binder.

According to a preferred embodiment, the viscosity of the composition is less than 50,000 Pa*s, preferably less than 40,000 Pa*s, more preferably from 1,000 to 30,000 Pa*s, in a sub-range of the temperature interval between 60 and 140°C. In this temperature interval the composition is in the state of a melt, which can be shaped. Generally, most glasses encompassed by the present invention have a suitable viscosity for bringing into the desired geometry at 20 to 30°C or even approximately 40°C above Tg of the binder. The viscosity of the composition is very important during injection of the melt into the moulding compartment and furthermore in the embodiments where the therapeutic agent is mixed with the melted binder.

As described above the composition may be provided with different dissolution rates according to the type of binder.

The melt consisting of the ingredients for the pharmaceutical composition may be prepared by mixing the at least one therapeutic agent homogeneously with a binder, obtaining melt matrix.

The exact order of the steps may be varied. The melt matrix constituting the binder may be produced in larger quantities and stored for later processing or disintegrated and stored, or processed immediately.

Different methods for producing the melt matrix may also be employed. The binder can be melted and quenched to make the amorphous glass matrix. The binder can be dissolved in a suitable solvent, preferentially in water, and the solvent removed by various methods such as boiling, vacuum boiling, freeze drying, spray drying, vacuum evaporation, air drying, or fluidised bed drying. Upon removal of the solvent, the binder will form matrix for further processing.

Similarly, the therapeutic agent may be added at different points of the process. Preferentially it is added when the binder is in the state of a dry powder as mentioned above, but it may also, where appropriate, be kneaded into the melted binder. It may also be added to a solution of the binder and be dried or melted together with the binder. The choice of method will depend primarily on the glass forming temperature of the binder and the ability of the therapeutic agent to withstand elevated temperatures.

In a preferred embodiment, the therapeutic agent is dissolved in a solution and then mixed with a solution of the binder. The mixed solutions may then be dried, shaped, optionally preceded by a drying step.

According to one embodiment, the binder is brought into a solid amorphous glass matrix by dissolving in a solvent and drying, preferentially by freeze-drying. After preparing the amorphous glass matrix, the matrix can be processed into a powder by known methods.

According to an especially preferred embodiment, the binder and the at least one therapeutic agent are mixed homogeneously as powders. An advantage of this embodiment is that the therapeutic agent and the binder can be mixed in an essentially dry state where both ingredients are in the shape of a powder. Mixing under these conditions is performed very easily. Experiments have shown that it may be difficult to obtain a homogeneous mixture when the therapeutic agent is added to the binder in a molten condition and for instance kneaded into the binder at least when produced in small amounts. An additional advantage of mixing in the dry state is that mixing can take place at ambient temperature whereby the activity of the therapeutic agent is conserved. When the composition has been mixed, it is melted and can immediately be injection moulded whereby prolonged exposure of the therapeutic agent to elevated temperatures is avoided.

When referring herein to preferred dimensions of the pharmaceutical composition the diameter of a substantially circular rod is used as a measure for the cross section area. For the triangular or otherwise formed rods the cross section area is correlated to the diameter of a corresponding circular rod. The diameter of the elongated pharmaceutical composition is preferably in the range of 0.2 to 1.0 mm, such as more preferred in the range of 0.3 to 0.7, even more preferred in the range of 0.4 to 0.6 mm. The diameter of the pharmaceutical composition is important with respect to the pain associated with the injection of the formulation, the smaller diameter the better. However, in order to obtain a sufficient amount of drug in the formulation to be injected, it is important that the diameter is not too small. By providing the pharmaceutical composition with this thickness, it has been determined that it can be injected essentially without pain. A further advantage is that less force is required to penetrate the skin as the diameter is reduced. By needles formed of the pharmaceutical composition according to the invention, it has been found that even at these dimensions they still have the necessary strength to penetrate the cutis or mucosa upon injection. A further advantage of using small diameters is that the surface area to volume ratio is higher than for larger diameters. Thereby the pharmaceutical compositions are dissolved more rapidly and the drugs can enter the body fluids to exert their effect. However, a too small diameter will require a very long pharmaceutical composition in order to contain the predetermined amount of therapeutic agent. A too small diameter would also reduce the compressive strength of the pharmaceutical composition and maybe cause it to break upon injection.

In practice the length of the pharmaceutical composition is largely determined by the dose of the therapeutic agent, the amount of binder, and the selected diameter. The dose of many therapeutic proteins is approximately 1 mg. One mg of protein excluding binder corresponds approximately to a cylinder with a diameter of 0.5 mm and a length of 3 mm. If such a pharmaceutical composition containing 1 mg of protein is made from 50 % therapeutic agent and 50 % binder, the pharmaceutical composition has a length of 6 mm. When the required dose is smaller, the dimension of the pharmaceutical composition will be reduced accordingly. A dose of 1/3 mg protein in a pharmaceutical composition with 50 % binder having a diameter of 0.5 mm has an approximate length of 2 mm. The invention is not restricted to any specific volume, the volume being determined by the length and diameter of the pharmaceutical composition. In most cases, the volume of the pharmaceutical composition is less than 5 µl, preferably less than 1 µl. Volumes down to 0.25 µl can obtained for small doses of therapeutic agent. Thus, the above-mentioned pharmaceutical composition having a diameter of 0.5 mm and a length of 2 mm has a volume of 0.39 µl.

Thus the length of the pharmaceutical composition normally is in the range of from 0.05 mm to 30 mm, such as from 0.1 mm to 20 mm, such as from 1.0 mm to 10 mm.

Although the pharmaceutical composition itself has a very small cross section, it has been shown that the pain associated with injection of the pharmaceutical composition decreases when the pharmaceutical composition comprises a pointed end. As a consequence, the pharmaceutical composition is preferably moulded with a pointed end.

As a human skin model, porcine abdomen skin has been used in penetration tests. Graphite rods with differently shaped pointed ends are pressed into porcine skin with a Lloyd Instrument LR5K, UK. The pressure force is measured in Newton as a function of the distance. The maximum force is used to compare the different rod shapes. No point (180°) on the rod is unsatisfactory and the rod may break before entering the skin. Using a graphite rod with a cone shaped point (90° top angle) is sufficient to penetrate the skin. However, a top angle of 60° significantly improves the penetration of the skin. Points with an angle below 20° are often very thin and thereby fragile.

Accordingly, the pointed end tapers preferably into an acute angle, wherein the angle is in the range between 10° to 110 °, preferably less than 90 °, more preferably less than 60 °. The pointed end may assume any configuration depending of the shape of the pharmaceutical composition itself, thus for a substantially circular pharmaceutical composition the pointed end may have the shape of a cone, whereas a pharmaceutical composition having a square cross section has a pyramid-formed pointed end. In a more preferred embodiment the top angle of the pointed end should be between 20 and 110°, preferably below 90°, more preferably below 70°.

The pointed end may be located in any suitable relation to the longitudinal axis of the pharmaceutical composition, such as centered or eccentric.

The pharmaceutical composition may be defined by a cylindric part and a pointed-end part. In this case another way of defining the pointed end is the reduction of the diameter from the beginning of the pointed-end part, i.e. towards the cylindrical part of the formulation, to the most tapered end of the pointed end. It is preferred that the diameter is reduced by at least 30 %, such as at least 40 %. Independent of the reduction it is preferred that the pointed end is rounded.

Any type of therapeutic agent can be incorporated into the pharmaceutical composition and the invention is not limited to drugs with any specific function. Thus the therapeutic agent may be selected from analgesics, antianxiety drugs, antiarthritic drugs, antibiotic agents, anticholinergics, antidepressants, antidiabetics, antiemetics, antihistaminics, antihypertensive agents, antiinflammatory drugs, antimigraine agents, antiparkinsonism agents, antispasmodesics, antipsychotics, antithrombotic agents, antiviral agents, appetite suppressants, blood factors, cardiovascular drugs, cerebral vasodilators, chemotherapeutic drugs, cholinergic agonists, contraceptives, coronary agents, diuretics, growth factors, coagulation factors, hormonal agents, immunosuppressive agents, narcotic antagonists, opiods, peripheral asodilators, tranquilizers, vaccines, immunogenic agents, and immunising agents.

Similarly, the therapeutic agent may be any type of compound such as steroids, hormones, lipids, nucleic acids, nucleotides, oligonucleotides, oligosaccharides, organics, antibodies, peptide mimetics, peptides, polypeptides, polysaccharides, and proteins. In particular the therapeutic agent may be a peptide, a polypeptide or a protein. Actually, the pharmaceutical composition may also contain subcellular pharmaceutical compositions, cells, bacteria or vira as a therapeutic agent for immunogenic purposes.

For some purposes it is important that the therapeutic agent is homogeneously distributed throughout the pharmaceutical composition so that its release is initiated as soon as the pharmaceutical composition starts dissolving.

According to a preferred embodiment, the therapeutic agent is selected from hormones, antidiabetic drugs, growth factors, and blood factors. Preferably, the therapeutic agent is a protein selected from the group insulin, glucagon, growth hormone, growth factors, blood factors such as FVII or FVIII, GLP-1, EPO, TPO, interferon or derivatives of these proteins. Such proteins can either be naturally occurring proteins or recombinant proteins.

The pharmaceutical composition may be produced from the therapeutic agent alone, it will however be appropriate to include a binder in the formulation. Any suitable binder may be used as discussed above.

Apart from the binder and the therapeutic agent, the pharmaceutical composition may comprise additives, which could be selected from but is not restricted to the group of preservatives, stabilisers, adjuvants, lubricants, and disintegraters. Some therapeutic agents may need to be preserved or stabilised through the use of a preservative or stabiliser, although this is likely to be necessary only in a few cases, owing to the almost anhydrous conditions in the pharmaceutical composition. In the cases where the therapeutic agent is for immunisation, it may be preferential to add an adjuvant to increase the immunogenic response. Lubricants such as fatty acids or their salts may be added to ensure that the pharmaceutical composition does not stick to the packaging and/or to provide lubrication as the pharmaceutical composition penetrates the skin. Lubricants may be stearates, such as Mg-stearates, Zn-stearates or Ca-stearates. In cases where a rapid release of the therapeutic agent is desired and in cases where the therapeutic agent comprises a large proportion of the pharmaceutical composition it may be necessary to add disintegrators which will cause the pharmaceutical composition to disintegrate and thereby release the therapeutic agent rapidly.

Also the pharmaceutical composition may comprise stabilizers, such as alanine, histidine and glycine.

In addition, the therapeutic agent of the pharmaceutical composition according to the invention is long term stable even at ambient temperature and there is no need for special storage conditions such as refrigeration. Furthermore, the pharmaceutical composition is stable at ambient temperature both in terms of the compressive strength, the glassy nature of the binder and the geometry.

The pharmaceutical composition can preferably be used for patients requiring frequent medication such as diabetics. By frequent is meant that the therapeutic agent must be injected parenterally at least once a day. Such patients always need to carry with them a quantity of therapeutic agent for injection. The convenience of administration as well as the convenience of storage of the pharmaceutical compositions according to the present invention makes it especially useful for this group of patients.

Another preferred use of the pharmaceutical composition is for immunisation. Immunisation of children is often carried out in the clinics of general practitioners that will appreciate the less rigid storage requirements of the pharmaceutical compositions according to the inventions. The same cartridge containing several pharmaceutical compositions can be used for different children, since there is no risk for cross contamination. The only object that penetrates the skin of the patient is the pharmaceutical composition itself. The injection will not cause the injection device or the cartridge housing the pharmaceutical compositions to be contaminated. Additionally, children who often suffer from pre-injection fear will appreciate the almost painless injection that can be carried out.

Another large group of patients requiring immunisation is in the tropics and during epidemics where large groups of individuals need immunisation at essentially the same time. Using the pharmaceutical compositions according to the present invention for mass immunisations is much more rapid and much safer than using conventional injection of aqueous solutions or suspensions of the immunoactive agent. Mass medication is also frequently used in animal and fish farming. In these cases it will also be of great advantage to use the pharmaceutical compositions according to the present invention for reasons of speed and reduction of cross contamination.

The pharmaceutical composition may be injected for localisation in dermis or further into subcutis or maybe even to more profound layers.

The invention further relates to a cassette comprising a packaging material as defined above and furthermore comprising ejecting means for ejecting the pharmaceutical composition from the cavity.

Any suitable ejecting means may be provided. In one embodiment the ejecting means is a spring-loaded hammer means. The ejecting means is suitable arranged to exert an ejecting pressure on a pin arranged in one end of the cavity adjacent the pharmaceutical composition.

Cassettes comprising more units can be shaped as beams, cylinders or bands which can be mounted in an insertion position in which a hammer can be released to hit the pin of the unit in question to move the pharmaceutical composition out of the packaging material into the skin, so that the pin functions as an anvil.

The pharmaceutical packaging material may be used in a method of administering a dosage of therapeutic agent comprised in a pharmaceutical composition to a patient in need thereof from a cassette as defined above, comprising
arranging the cassette adjacent the skin of the patient,
activating an ejecting means,
injecting the pharmaceutical composition comprising the therapeutic agent.

By this administration form the pharmaceutical composition is administrated in a simple, substantially painless and sterile manner without the use of any cannulas.

In another embodiment the cassette is provided with a cannula whereby the cannula penetrates the skin or mucosa and the pharmaceutical composition is pressed into the dermis or subcutis of the patient through the cannula.

## Claims

1. A method of producing a pharmaceutical composition in a packaging material comprising the steps of
establishing a mould,
moulding, in said mould, the packaging material from a melt, whereby the packaging material is provided with at least one cavity, said cavity having at least one side wall and optionally a bottom wall,
moulding in a moulding compartment the pharmaceutical composition from a pharmaceutical melt consisting of the ingredients of the pharmaceutical composition having a predetermined temperature, wherein said moulding compartment having a predetermined form being defined by at least a part of said at least one cavity,
cooling the pharmaceutical composition,
obtaining in the packaging material comprising the pharmaceutical composition having a predetermined form.

2. The method according to claim 1, wherein the cavity is an elongated cavity.

3. The method according to claim 1 or 2, wherein the cavity side wall is defined by the packaging material.

4. The method according to any of the preceding claims, wherein the moulding compartment is further defined by an insertion means arranged in an open end of the cavity.

5. The method according to claim 4, wherein the insertion means is provided with an injection head for injecting the pharmaceutical composition melt into the moulding compartment.

6. The method according to any of the preceding claims 2-5, wherein the predetermined form of the pharmaceutical composition is an elongated form.

7. The method according to claim 6, wherein one end of the elongated pharmaceutical composition is plane.

8. The method according to claim 6 or 7, wherein one end of the elongated pharmaceutical composition is moulded with a tip.

9. The method according to claim 8, wherein the tip is defined by a tip mould insert arranged in an open end of the cavity.

10. The method according to claim 8, wherein the tip mould insert is arranged in the end of the cavity opposite the insertion means comprising the injection head.

11. The method according to claim 8, wherein the cavity is provided with a bottom wall and the tip is defined by the bottom wall.

12. The method according to any of the preceding claims, wherein the packaging material is moulded from a polymer melt.

13. The method according to claim 12, wherein the moulded packaging material is an amorphous polymer material.

14. The method according to claim 13, wherein the amorphous polymer material is a cyclic olefin copolymer.

15. The method according to any of the preceding claims, wherein the glass transition temperature (Tg) of the packaging material is above 100 °C.

16. The method according to any of the preceding claims, further comprising a cooling step comprising cooling the moulded packaging material before moulding the pharmaceutical composition.

17. The method according to claim 16, comprising cooling the moulded packaging material to a temperature substantially identical to the predetermined temperature of the pharmaceutical composition melt.

18. The method according to any of the preceding claims, wherein the moulded packaging material contracts at most 5 % after the step of moulding the pharmaceutical composition.

19. The method according to any of the preceding claims, wherein the packaging material is comprising at least 5 cavities arranged in parallel, said cavities being substantially identical.

20. The method according to claim 19, wherein the pharmaceutical compositions of each cavity are moulded simultaneously.

21. The method according to any of the preceding claims 1-18, wherein the packaging material is comprising one cavity.

22. The method according to claim 21, further comprising a step of assembling two or more packaging materials through a hinging means.

23. The method according to any of the preceding claims, further comprising moulding a pin being dimensioned to fit into the open end of the cavity after retracting an insertion means.

24. The method according to claim 23, wherein the pin is moulded with the packaging material.

25. A moulded packaging material comprising
- at least one cavity, said cavity having at least one side wall and a bottom wall,
- and a pharmaceutical composition having an elongated form and being moulded in at least a part of said at least one cavity from a melt consisting of the ingredients of the pharmaceutical composition, whereby the pharmaceutical composition is in contact with the cavity side wall in an area being more than half of the surface area of the pharmaceutical composition, and
wherein the bottom wall functions as a diffusion tight sealing of one end of the cavity, said sealing being moulded from packaging material melt.

26. The packaging material according to claim 25, wherein the cavity is an elongated cavity.

27. The packaging material according to claim 25 or 26, wherein the cavity side wall is defined by the packaging material.

28. The packaging material according to claim 27, wherein one end of the elongated pharmaceutical composition is plane.

29. The packaging material according to claim 27 or 28, wherein one end of the elongated pharmaceutical composition is provided with a tip.

30. The packaging material according to claim 29, wherein the top angle of the tip is in the range of 10°C-110°C.

31. The packaging material according to claim 25, wherein the sealing is shaped to correspond to the tip of the pharmaceutical composition.

32. The packaging material according to any of the preceding claims 25-31, further comprising a pin being dimensioned to fit into the open end of the cavity.

33. The packaging material according to claim 32, wherein the pin is moulded with the packaging material.

34. The packaging material according to claim 32 or 33, wherein the pin is providing a diffusion tight sealing of the cavity.

35. The packaging material according to any of the preceding claims 25-34, wherein the packaging material is moulded from a polymer melt.

36. The packaging material according to claim 35, wherein the moulded packaging material is an amorphous polymer material.

37. The packaging material according to claim 36, wherein the amorphous polymer material is a cyclic olefin copolymer.

38. The packaging material according to any of the preceding claims 25-37, wherein the glass transition temperature (Tg) of the packaging material is above 100 °C.

39. The packaging material according to any of the preceding claims 25-38, wherein the moulded packaging material contracts at most 5 % after moulding the pharmaceutical composition therein.

40. The packaging material according to any of the preceding claims 25-39, wherein the packaging material is comprising at least 5 cavities arranged in parallel, said cavities being substantially identical.

41. The packaging material according to any of the preceding claim 25-40, comprising at least 5 cavities each being provided with a pharmaceutical composition.

42. The packaging material according to claim 41, wherein the packaging material comprises a number of cavities being hinged together forming a cartridge band comprising several cavities.

43. The packaging material according to any of the preceding claims 25-42, wherein the pharmaceutical composition comprises a therapeutic agent selected from the group consisting of analgesics, antianxiety drugs, antiarthritic drugs, antibiotic agents, anticholinergics, antidepressants, antidiabetics, antiemetics, antihistaminics, antihypertensive agents, antiinflammatory drugs, antimigraine agents, antiparkinsonism agents, antispasmodesics, antipsychotics, antithrombotic agents, antiviral agents, appetite suppressants, blood factors, cardiovascular drugs, cerebral vasodilators, chemotherapeutic drugs, cholinergic agonists, contraceptives, coronary agents, diuretics, hormonal agents, immunosuppressive agents, growth factors, narcotic antagonists, opiods, peripheral asodilators, tranquilizers, vaccines, immunogenic agents, and immunising agents.

44. The packaging material according to any of the preceding claims 25-42, wherein the therapeutic agent is selected from hormones, lipids, nucleic acids, nucleotides, oligonucleotides, oligosaccharides, organics, peptide mimetics, antibodies, peptides, polysaccharides, and proteins.

45. The packaging material according to any of the preceding claims 25-42, wherein the therapeutic agent is selected from proteins, peptides, and polypeptides, said protein, peptide, or polypeptide being amorphous or crystalline.

46. The packaging material according to any of the preceding claims 43-45, wherein the therapeutic agent is selected from hormones, antidiabetic drugs, growth factors, and blood factors, preferably being a protein selected from insulin, glucagon, growth hormone, blood factor such as FVII and FVIII, GLP-1, EPO, TPO, interferon or derivatives of these proteins.

47. A cassette comprising a packaging material as defined in any of the claims 25-46 and furthermore comprising ejecting means for ejecting the pharmaceutical composition from the cavity.

48. The cassette according to claim 47, wherein the ejecting means is a spring-loaded hammer means.

49. The cassette according to claim 47 or 48, wherein the ejecting means is arranged to exert an ejecting pressure on a pin arranged in one end of the cavity adjacent to the pharmaceutical composition.

## Patentansprüche

1. Verfahren zur Herstellung eines Arzneimittels in einem Verpackungsmaterial, umfassend die Schritte
Festlegen einer Gussform,
Gießen des Verpackungsmaterials in der Gussform aus einer Schmelze, wodurch das Verpackungsmaterial mit mindestens einem Hohlraum versehen wird, wobei der Hohlraum mindestens eine Seitenwand und wahlweise eine Bodenwand aufweist,
Gießen des Arzneimittels in einer Gussabteilung aus einer Arzneimittelschmelze, bestehend aus den Inhaltsstoffen des Arzneimittels mit einer vorbestimmten Temperatur, wobei die Gussabteilung eine vorbestimmte Form aufweist, die durch mindestens einen Teil des mindestens einen Hohlraums definiert ist,
Abkühlen des Arzneimittels,
Erhalt des Arzneimittels im Verpackungsmaterial mit einer vorbestimmten Form.

2. Verfahren nach Anspruch 1, wobei der Hohlraum ein länglicher Hohlraum ist.

3. Verfahren nach Anspruch 1 oder 2, wobei die Hohlraumseitenwand durch das Verpackungsmaterial definiert ist.

4. Verfahren nach einem der vorangehenden Ansprüche, wobei die Gussabteilung ferner durch ein in einem offenen Ende des Hohlraums angeordnetes Einführungsmittel definiert ist.

5. Verfahren nach Anspruch 4, wobei das Einführungsmittel mit einem Spritzkopf zum Einspritzen der Arzneimittelschmelze in die Gussabteilung versehen ist.

6. Verfahren nach einem der vorangehenden Ansprüche 2-5, wobei die vorbestimmte Form des Arzneimittels eine längliche Form ist.

7. Verfahren nach Anspruch 6, wobei ein Ende des länglichen Arzneimittels flach ist.

8. Verfahren nach Anspruch 6 oder 7, wobei ein Ende des länglichen Arzneimittels mit einer Spitze gegossen ist.

9. Verfahren nach Anspruch 8, wobei die Spitze durch einen in einem offenen Ende des Hohlraums angeordneten Spitzengusseinsatz definiert ist.

10. Verfahren nach Anspruch 8, wobei der Spitzengusseinsatz im Ende des Hohlraums gegenüber dem den Spritzkopf umfassenden Einführungsmittel angeordnet ist.

11. Verfahren nach Anspruch 8, wobei der Hohlraum mit einer Bodenwand versehen ist und die Spitze durch die Bodenwand definiert ist.

12. Verfahren nach einem der vorangehenden Ansprüche, wobei das Verpackungsmaterial aus einer Polymerschmelze gegossen ist.

13. Verfahren nach Anspruch 12, wobei das Verpackungsgussmaterial ein amorphes Polymermaterial ist.

14. Verfahren nach Anspruch 13, wobei das amorphe Polymermaterial ein cyclisches Olefin-Copolymer ist.

15. Verfahren nach einem der vorangehenden Ansprüche, wobei die Glasübergangstemperatur (Tg) des Verpackungsmaterials über 100°C liegt.

16. Verfahren nach einem der vorangehenden Ansprüche, ferner umfassend einen Abkühlschritt, umfassend das Abkühlen des Verpackungsgussmaterials vor dem Gießen des Arzneimittels.

17. Verfahren nach Anspruch 16, umfassend das Abkühlen des Verpackungsgussmaterials auf eine Temperatur, die im Wesentlichen mit der vorbestimmten Temperatur der Arzneimittelschmelze identisch ist.

18. Verfahren nach einem der vorangehenden Ansprüche, wobei sich das Verpackungsgussmaterial nach dem Schritt des Gießens des Arzneimittels um höchstens 5% zusammenzieht.

19. Verfahren nach einem der vorangehenden Ansprüche, wobei das Verpackungsmaterial mindestens 5 parallel angeordnete Hohlräume umfasst, wobei die Hohlräume im Wesentlichen identisch sind.

20. Verfahren nach Anspruch 19, wobei die Arzneimittel von jedem Hohlraum gleichzeitig gegossen werden.

21. Verfahren nach einem der vorangehenden Ansprüche 1-18, wobei das Verpackungsmaterial einen Hohlraum umfasst.

22. Verfahren nach Anspruch 21, ferner umfassend einen Schritt des Zusammenschließens von zwei oder mehreren Verpackungsmaterialien durch ein Scharniermittel.

23. Verfahren nach einem der vorangehenden Ansprüche, ferner umfassend das Gießen eines Stifts, der derart bemessen ist, dass er nach Zurückziehen eines Einführungsmittels in das offene Ende des Hohlraums passt.

24. Verfahren nach Anspruch 23, wobei der Stift mit dem Verpackungsmaterial gegossen wird.

25. Verpackungsgussmaterial, umfassend
- mindestens einen Hohlraum, wobei der Hohlraum mindestens eine Seitenwand und eine Bodenwand aufweist,
- und ein Arzneimittel mit einer länglichen Form, das in mindestens einem Teil des mindestens einen Hohlraums aus einer aus den Inhaltsstoffen des Arzneimittels bestehenden Schmelze gegossen ist6, wobei das Arzneimittel mit der Hohlraumseitenwand in einem mehr als die Hälfte des Oberflächenbereichs des Arzneimittels ausmachenden Bereich in Kontakt ist, und
wobei die Bodenwand als diffusionsdichte Versiegelung eines Endes des Hohlraums fungiert, wobei die Versiegelung aus Verpackungsmaterialschmelze gegossen ist.

26. Verpackungsmaterial nach Anspruch 25, wobei der Hohlraum ein länglicher Hohlraum ist.

27. Verpackungsmaterial nach Anspruch 25 oder 26, wobei die Hohlraumseitenwand durch das Verpackungsmaterial definiert ist.

28. Verpackungsmaterial nach Anspruch 27, wobei ein Ende des länglichen Arzneimittels flach ist.

29. Verpackungsmaterial nach Anspruch 27 oder 28, wobei ein Ende des länglichen Arzneimittels mit einer Spitze versehen ist.

30. Verpackungsmaterial nach Anspruch 29, wobei der obere Winkel der Spitze im Bereich von 10° - 110° liegt.

31. Verpackungsmaterial nach Anspruch 25, wobei die Versiegelung derart gestaltet ist, dass sie der Spitze des Arzneimittels entspricht.

32. Verpackungsmaterial nach einem der vorangehenden Ansprüche 25-31, ferner umfassend einen Stift der derart bemessen ist, dass er in das offene Ende des Hohlraums passt.

33. Verpackungsmaterial nach Anspruch 32, wobei der Stift mit dem Verpackungsmaterial gegossen ist.

34. Verpackungsmaterial nach Anspruch 32 oder 33, wobei der Stift eine diffusionsdichte Versiegelung des Hohlraums bereitstellt.

35. Verpackungsmaterial nach einem der vorangehenden Ansprüche 25-34, wobei das Verpackungsmaterial aus einer Polymerschmelze gegossen ist.

36. Verpackungsmaterial nach Anspruch 35, wobei das Verpackungsgussmaterial ein amorphes Polymermaterial ist.

37. Verpackungsmaterial nach Anspruch 36, wobei das amorphe Polymermaterial ein cyclisches Olefin-Copolymer ist.

38. Verpackungsmaterial nach einem der vorangehenden Ansprüche 25-37, wobei die Glasübergangstemperatur (Tg) des Verpackungsmaterials über 100°C liegt.

39. Verpackungsmaterial nach einem der vorangehenden Ansprüche 25-38, wobei sich das Verpackungsgussmaterial nach dem Formen des Arzneimittels um höchstens 5% darin zusammenzieht.

40. Verpackungsmaterial nach einem der vorangehenden Ansprüche 25-39, wobei das Verpackungsmaterial mindestens 5 parallel angeordnete Hohlräume umfasst, wobei die Hohlräume im Wesentlichen identisch sind.

41. Verpackungsmaterial nach einem der vorangehenden Ansprüche 25-40, umfassend mindestens 5 Hohlräume, die jeweils mit einem Arzneimittel versehen sind.

42. Verpackungsmaterial nach Anspruch 41, wobei das Verpackungsmaterial eine Anzahl von Hohlräumen umfasst, die unter Bildung eines Patronenbands, umfassend mehrere Hohlräume, miteinander scharnierartig verbunden sind.

43. Verpackungsmaterial nach einem der vorangehenden Ansprüche 25-42, wobei das Arzneimittel ein Therapeutikum umfasst, ausgewählt aus der Gruppe bestehend aus Analgetika, Arzneimitteln gegen Angst, Mitteln gegen Arthritis, Antibiotika, Anticholinergika, Antidepressiva, Antidiabetika, Antiemetika, Antihistaminika, Mitteln gegen Bluthochdruck, entzündungshemmenden Mitteln, Mitteln gegen Migräne, Mitteln gegen Parkinson-Krankheit, antispasmoiden Mitteln, Antipsychotika, Antithrombotika, antiviralen Mitteln, Appetitzüglern, Blutfaktoren, kardiovaskulären Mitteln, Cerebralvasodilatoren, Chemotherapeutika, cholinergen Agonisten, Kontrazeptiva, Koronärmitteln, Diuretika, Wachstumsfaktoren, Gerinnungsfaktoren, Hormonen, Immunsuppressiva, narkotischen Antagonisten, Opioiden, peripheren Vasodilatoren, Tranquilizern, Impfstoffen, immunogenen Mitteln und immunisierenden Mitteln.

44. Verpackungsmaterial nach einem der vorangehenden Ansprüche 25-42, wobei das Therapeutikum ausgewählt ist aus Hormonen, Lipiden, Nukleinsäuren, Nukleotiden, Oligonukleotiden, Oligosacchariden, organischen Verbindungen, Peptidmimetika, Antikörpern, Peptiden, Polysacchariden und Proteinen.

45. Verpackungsmaterial nach einem der vorangehenden Ansprüche 25-42, wobei das Therapeutikum ausgewählt ist aus Proteinen, Peptiden und Polypeptiden, wobei das Protein, Peptid oder Polypeptid amorph oder kristallin ist.

46. Verpackungsmaterial nach einem der vorangehenden Ansprüche 43-45, wobei das Therapeutikum ausgewählt ist aus Hormonen, Antidiabetika, Wachstumsfaktoren und Blutfaktoren, wobei es sich vorzugsweise um ein Protein handelt, ausgewählt aus Insulin, Glucagon, Wachstumshormon, Blutfaktor wie FVII und FVIII, GLP-1, EPO, TPO, Interferon oder Derivaten dieser Proteine.

47. Kassette, umfassend ein Verpackungsmaterial, wie in einem der Ansprüche 25-46 definiert, und weiterhin umfassend Auswurfmittel zum Auswerfen des Arzneimittels aus dem Hohlraum.

48. Kassette nach Anspruch 47, wobei das Auswurfmittel ein feder-belastetes Hammermittel ist.

49. Kassette nach Anspruch 47 oder 48, wobei das Auswurfmittel derart angeordnet ist, dass es auf einen in einem Ende des Hohlraums neben dem Arzneimittel angeordneten Stift einen Auswurfdruck ausübt.

## Revendications

1. Procédé de production d'une composition pharmaceutique dans une matière d'emballage, le procédé comprenant les étapes de :
création d'un moule,
moulage, dans ledit moule, de la matière d'emballage à partir d'une matière fondue, la matière d'emballage étant alors pourvue d'au moins une cavité, ladite cavité présentant au moins une paroi latérale et, éventuellement, une paroi de fond,
moulage, dans un compartiment de moulage, de la composition pharmaceutique à partir d'une coulée de substances pharmaceutiques, composée des ingrédients de la composition pharmaceutique, et ayant une température prédéterminée, ledit compartiment de moulage présentant une forme prédéterminée, qui est définie par au moins une partie de ladite au moins une cavité,
refroidissement de la composition pharmaceutique,
obtention, dans la matière d'emballage, de la composition pharmaceutique ayant une forme prédéterminée.

2. Procédé selon la revendication 1, dans lequel la cavité est une cavité oblongue.

3. Procédé selon la revendication 1 ou 2, dans lequel la paroi latérale de la cavité est définie par la matière d'emballage.

4. Procédé selon l'une quelconque des revendications précédentes, dans lequel le compartiment de moulage est, en outre, défini par un moyen d'introduction, disposé dans une extrémité ouverte de la cavité.

5. Procédé selon la revendication 4, dans lequel le moyen d'introduction est pourvu d'une tête d'injection afin d'injecter la coulée de composition pharmaceutique dans le compartiment de moulage.

6. Procédé selon l'une quelconque des revendications 2 à 5, dans lequel la forme prédéterminée de la composition pharmaceutique est une forme oblongue.

7. Procédé selon la revendication 6, dans lequel une extrémité de la composition pharmaceutique oblongue est plane.

8. Procédé selon la revendication 6 ou 7, dans lequel une extrémité de la composition pharmaceutique est moulée de façon à présenter une pointe.

9. Procédé selon la revendication 8, dans lequel la pointe est définie par un insert de moulage de pointe, disposé dans une extrémité ouverte de la cavité.

10. Procédé selon la revendication 8, dans lequel l'insert de moulage de pointe est disposé dans l'extrémité de la cavité, à l'opposé du moyen d'introduction comprenant la tête d'injection.

11. Procédé selon la revendication 8, dans lequel la cavité est pourvue d'une paroi de fond, et la pointe est définie par la paroi de fond.

12. Procédé selon l'une quelconque des revendications précédentes, dans lequel la matière d'emballage est moulée à partir d'une coulée de matière polymère.

13. Procédé selon la revendication 12, dans lequel la matière moulée d'emballage est une matière polymère amorphe.

14. Procédé selon la revendication 13, dans lequel la matière polymère amorphe est un copolymère d'oléfine cyclique.

15. Procédé selon l'une quelconque des revendications précédentes, dans lequel la température de transition vitreuse (Tg) de la matière d'emballage est supérieure à 100 °C.

16. Procédé selon l'une quelconque des revendications précédentes, comprenant en outre une étape de refroidissement, pour le refroidissement de la matière moulée d'emballage avant le moulage de la composition pharmaceutique.

17. Procédé selon la revendication 16, comprenant le refroidissement de la matière moulée d'emballage jusqu'à une température sensiblement identique à la température prédéterminée de la coulée de composition pharmaceutique.

18. Procédé selon l'une quelconque des revendications précédentes, dans lequel la matière moulée d'emballage présente une retrait de 5 % au maximum après l'étape de moulage de la composition pharmaceutique.

19. Procédé selon l'une quelconque des revendications précédentes, dans lequel la matière d'emballage comprend au moins 5 cavités disposées en parallèle, lesdites cavités étant sensiblement identiques.

20. Procédé selon la revendication 19, dans lequel les compositions pharmaceutiques sont moulées simultanément dans chaque cavité.

21. Procédé selon l'une quelconque des revendications précédentes 1 à 18, dans lequel la matière d'emballage comporte une seule cavité.

22. Procédé selon la revendication 21, comprenant en outre une étape d'assemblage de deux matières d'emballage ou plus, par l'intermédiaire d'un moyen d'articulation.

23. Procédé selon l'une quelconque des revendications précédentes, comprenant en outre le moulage d'une tige, qui est dimensionnée pour s'adapter dans l'extrémité ouverte de la cavité après qu'un moyen d'introduction a été retiré.

24. Procédé selon la revendication 23, dans lequel la tige est moulée d'une seule pièce avec la matière d'emballage.

25. Matière moulée d'emballage, comprenant :
- au moins une cavité, ladite cavité présentant au moins une paroi latérale et une paroi de fond,
- et une composition pharmaceutique, présentant une forme oblongue et moulée dans au moins une partie de ladite au moins une cavité, à partir d'une coulée composée des ingrédients de la composition pharmaceutique, la composition pharmaceutique étant alors en contact avec la paroi latérale de la cavité dans une zone ne représentant pas plus de la moitié de la superficie de la composition pharmaceutique, et
dans laquelle la paroi de fond fait fonction de fermeture étanche à une diffusion, d'une extrémité de la cavité, ladite fermeture étanche étant moulée à partir de la coulée de matière d'emballage.

26. Matière d'emballage selon la revendication 25, dans laquelle la cavité est une cavité oblongue.

27. Matière d'emballage selon la revendication 25 ou 26, dans laquelle la paroi latérale de la cavité est définie par la matière d'emballage.

28. Matière d'emballage selon la revendication 27, dans laquelle une extrémité de la composition pharmaceutique oblongue est plane.

29. Matière d'emballage selon la revendication 27 ou 28, dans laquelle une extrémité de la composition pharmaceutique oblongue est pourvue d'une pointe.

30. Matière d'emballage selon la revendication 29, dans laquelle l'angle au sommet de la pointe est compris dans la fourchette de 10 °C à 110 °C.

31. Matière d'emballage selon la revendication 25, dans laquelle la fermeture étanche est conformée de manière à correspondre à la pointe de la composition pharmaceutique.

32. Matière d'emballage selon l'une quelconque des revendications précédentes 25 à 31, comprenant en outre une tige, qui est dimensionnée pour s'adapter dans l'extrémité ouverte de la cavité.

33. Matière d'emballage selon la revendication 32, dans laquelle la tige est moulée d'une seule pièce avec la matière d'emballage.

34. Matière d'emballage selon la revendication 32 ou 33, dans laquelle la tige constitue une fermeture étanche à la diffusion, pour la cavité.

35. Matière d'emballage selon l'une quelconque des revendications précédentes 25 à 34, dans laquelle la matière d'emballage est moulée à partir d'une coulée de matière polymère.

36. Matière d'emballage selon la revendication 35, dans laquelle la matière moulée d'emballage est une matière polymère amorphe.

37. Matière d'emballage selon la revendication 36, dans laquelle la matière polymère amorphe est un copolymère d'oléfine cyclique.

38. Matière d'emballage selon l'une quelconque des revendications précédentes 25 à 37, dans laquelle la température de transition vitreuse (Tg) de la matière d'emballage est supérieure à 100 °C.

39. Matière d'emballage selon l'une quelconque des revendications précédentes 25 à 38, dans laquelle la matière moulée d'emballage présente un retrait de 5 % au maximum après le moulage de la composition pharmaceutique dans celle-ci.

40. Matière d'emballage selon l'une quelconque des revendications précédentes 25 à 39, dans laquelle la matière d'emballage comprend au moins 5 cavités disposées en parallèle, lesdites cavités étant sensiblement identiques.

41. Matière d'emballage selon l'une quelconque des revendications précédentes 25 à 40, comprenant au moins 5 cavités, dont chacune est pourvue d'une composition pharmaceutique.

42. Matière d'emballage selon la revendication 41, dans laquelle la matière d'emballage comprend un certain nombre de cavités, qui sont reliées de manière articulée pour constituer une bande de cartouches comprenant plusieurs cavités.

43. Matière d'emballage selon l'une quelconque des revendications précédentes 25 à 42, dans laquelle la composition pharmaceutique comprend un agent thérapeutique, sélectionné parmi les analgésiques, les anxiolytiques, les médicaments antiarthritiques, les agents antibiotiques, les anticholinergiques, les antidépresseurs, les antidiabétiques, les antiémétiques, les antihistaminiques, les antihypertenseurs, les anti-inflammatoires, les agents antimigraine, les agents antiparkinsoniens, les antispasmodiques, les antipsychotiques, les agents antithrombose, les agents antiviraux, les anorexigènes, les facteurs sanguins, les médicaments cardio-vasculaires, les vasodilatateurs cérébraux, les médicaments chimiothérapiques, les agonistes cholinergiques, les contraceptifs, les agents coronariens, les diurétiques, les agents hormonaux, les immunosuppresseurs, les facteurs de croissance, les antagonistes de narcotiques, les opiacés, les vasodilatateurs périphériques, les tranquillisants, les vaccins, les agents immunogènes, et les agents d'immunisation.

44. Matière d'emballage selon l'une quelconque des revendications précédentes 25 à 42, dans laquelle l'agent thérapeutique est sélectionné parmi les hormones, les lipides, les acides nucléiques, les nucléotides, les oligonucléotides, les oligosaccharides, les substances organiques, les peptides mimétiques, les anticorps, les peptides, les polysaccharides, et les protéines.

45. Matière d'emballage selon l'une quelconque des revendications précédentes 25 à 42, dans laquelle l'agent thérapeutique est sélectionné parmi les protéines, les peptides et les polypeptides, ladite protéine, ledit peptide ou polypeptide étant amorphe ou cristallin(e).

46. Matière d'emballage selon l'une quelconque des revendications précédentes 43 à 45, dans laquelle l'agent thérapeutique est sélectionné parmi les hormones, les médicaments antidiabétiques, les facteurs de croissance, et les facteurs sanguins et il s'agit, de préférence, d'une protéine sélectionnée parmi l'insuline, le glucagon, l'hormone de croissance, un facteur sanguin, tel que FVII ou FVIII, le GLP-1, l'EPO, le TPO, l'interféron ou des dérivés de ces protéines.

47. Cassette comprenant une matière d'emballage selon l'une quelconque des revendications 25 à 46, et comprenant en outre un moyen d'éjection pour éjecter la composition pharmaceutique à partir de la cavité.

48. Cassette selon la revendication 47, dans laquelle le moyen d'éjection est un moyen percuteur à ressort.

49. Cassette selon la revendication 47 ou 48, dans laquelle le moyen d'éjection est prévu pour exercer une force d'éjection sur une tige, disposée dans une extrémité de la cavité, de manière adjacente à la composition pharmaceutique.
